# EUROPEAN PATENT APPLICATION

(11) **EP 4 541 351 A1**
(43) Date of publication of application: **23.04.2025**
(21) Application number: 23383060.3
(22) Date of filing: 18.10.2023
(51) Int. Cl.: A61K 31/09, A61K 31/357, A61P 19/02, A61P 19/04

(54) **COMPOUNDS AND COMBINATION AS STIMULATORS OF COLLAGEN SYNTHESIS**

(71) Applicant: Center for Intelligent Research in Crystal Engineering, S.L., 07121 Palma de Mallorca (ES)
(72) Inventor: ALBERTÍ PALMER, Juan Jesús, 07013 PALMA (ES); STELLA, Georgina Natalia, 07609 LLUCMAJOR (ES); PROHENS LÓPEZ, Rafel, 08201 SABADELL (ES); BARRERA BARCELÓ, Fernando, 07010 PALMA DE MALLORCA (ES)
(74) Representative: ZBM Patents - Zea, Barlocci & Markvardsen

(57) **Abstract**

The present disclosure relates to a compound selected from the group consisting of pterostilbene or a cocrystal thereof; silybin or a pharmaceutically acceptable salt of thereof, or any stereoisomer or mixtures of stereoisomers of any of them; a complex of silybin or of any of stereoisomer or mixtures of stereoisomers thereof; or a glycoside of silybin or of any stereoisomer or mixtures of stereoisomers thereof, either for use in the treatment of a disease or disorder mediated by the loss of endogenous type I collagen, or for use in joint functionality improvement, articular disease healing; tendon-bone, bone, and muscle repair improvement; or in wound healing. It also relates to combinations of pterostilbene or a cocrystal thereof, and silybin, particularly addressed to the treatment of a disease or disorder mediated by the loss of endogenous type I collagen, as well as to pharmaceutical compositions and kits containing them, and to the use of the latter in for any one of the mentioned treatments.

## Description

### Technical Field

The present disclosure relates to compounds and combinations capable of increase endogenous type I collagen production.

### Background Art

Collagen is a main protein constituting a connective tissue in animals, taking up nearly 30% of a total protein of a human body. Collagen is widely distributed in skin, cartilage tissues, a cornea, the heart, the liver or the like as a main component constituting a skeletal structure of a tissue form of an animal.

Fibril-forming collagens are by far the most abundant in nature. They comprise type I, type II, type III, type V, and XI. All these collagen types have in common the ability to assemble into a quarter-staggered fibril-array. Withing this group, type I and type II occur to a greater extend. Type I collagens represent more than 90% of organic matter in bone, dermis, tendon, ligaments, and cornea.

Biosynthesis of collagen has been observed in mesenchymal cell family (fibroblasts, chondroblasts or osteoblasts) as well as in other cell lines such as epithelial cells.

Since collagen specifically acts on adhesion of various kinds of cells, and differentiation or proliferation of a cell, and also has a role as a regulatory factor for cell function, decrease in collagen may cause various diseases, such as corneal disorders such as corneal ulcer, articular disorders such as rheumatism, arthritis, degenerative arthritis and osteoarthritis, and inflammatory diseases in some cases.

Particularly, type I collagen mediates functions such as joint functionality improvement, support in articular disease healing, tendon-bone, bone, and muscle repair improvement, supporting wound healing process, restoring of skin elasticity and tautness, reducing wrinkles or sagging of the skin, or ameliorating a corneal disorder.

Many of these conditions are addressed with topically or orally administered exogenous collagen. In some cases, as a matrix implant in an injured area (with the risk of generating a foreign object reaction). Additionally, it is not evident that orally ingested collagen reaches the right site unaltered to exert its effect. Moreover, administered collagen is usually animal derived, what has religious limitations, and may have risk of transmitting zoonotic diseases.

Therefore, there is a need of safe active agents having the ability for significantly enhancing the production of endogenous collagen, what would avoid the above mentioned problems.

### Summary of Invention

Inventors have surprisingly found that by administering pterostilbene, a cocrystal of pterostilbene and picolinic acid, silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof, endogenous type I collagen synthesis is increased. They have found that the mentioned compounds can be administered orally, parenterally, or topically. Particularly, topical administration allows to locally stimulate endogenous type I collagen synthesis.

As shown in the examples below, the inventors have found that the cocrystal of pterostilbene and picolinic acid (Pteroyouth^{®}), pterostilbene, and silybin promoted total collagen deposition in human fibroblasts.

Thus, the inventors have found that the administration of the abovementioned compounds allows treating a disease or disorder mediated by the loss of endogenous type I collagen.

Additionally, the stimulation of the synthesis of endogenous type I collagen allows achieving a joint functionality improvement; supports articular disease healing; tendon-bone, bone, and muscle repair improvement; and supports wound healing process.

Therefore, a first aspect of the present disclosure relates to a compound selected from the group consisting of:
- a cocrystal of pterostilbene and a coformer selected from the group consisting of picolinic acid, 1,4-dimethylpiperazine, 2,3,5-trimethylpyrazine, theophylline, ethylenediamine, 1,4-diazabicyclo[2.2.2]octane (DABCO), and 1,4,8,11-tetrazacyclohexandecane, 2,4-dihydroxybenzoic acid (2,4-DHBA), indole, lysine, orotic acid, phenanthroline, urea, L-arginine, glycine, caffeine, carbamazepine, piperazine, and glutaric acid;
- pterostilbene or a pharmaceutically acceptable salt thereof;
- silybin or a pharmaceutically acceptable salt of thereof, or any stereoisomer or mixtures of stereoisomers of any of them; a complex of silybin or of any of stereoisomer or mixtures of stereoisomers thereof; or a glycoside of silybin or of any stereoisomer or mixtures of stereoisomers thereof;
either for use in the treatment of a disease or disorder mediated by the loss of endogenous type I collagen, or for use in joint functionality improvement, articular disease healing; tendon-bone, bone, and muscle repair improvement; or in wound healing. [claim 1]

This aspect may also be formulated as the use of a compound selected from the group consisting of a cocrystal of pterostilbene and a coformer as defined above; pterostilbene or a pharmaceutically acceptable salt thereof; silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof; for the manufacture of a medicament for the treatment of a disease or disorder mediated by the loss of endogenous type I collagen. It also forms part of the invention a method for the treatment of a disease or disorder mediated by the loss of endogenous type I collagen, comprising administering to a subject in need thereof, including a human, a pharmaceutically effective amount of a cocrystal of pterostilbene and a coformer as defined above; pterostilbene or a pharmaceutically acceptable salt thereof; silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof; together with one or more pharmaceutically acceptable excipients or carriers.

Thus, a second aspect of the present disclosure relates to a pharmaceutical, cosmetical, or nutraceutical composition comprising a pharmaceutically, a cosmetically, or a nutraceutically effective amount of a compound as defined herein, together with one or more pharmaceutically, cosmetically, or nutraceutically acceptable excipients or carriers, for use in the treatment of a disease or disorder mediated by the loss of endogenous type I collagen, or for use in joint functionality improvement, articular disease healing; tendon-bone, bone, and muscle repair improvement; or in wound healing. [claim 6]
A third aspect of the present disclosure relates to a combination comprising: a) a compound selected from the group consisting of a cocrystal of pterostilbene and a coformer as defined above; and pterostilbene or a pharmaceutically acceptable salt thereof, and b) silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof [claim 9]
A fourth aspect of the present disclosure relates to a single pharmaceutical composition which comprises: a) a pharmaceutically effective amount of pterostilbene or a cocrystal thereof; b) a pharmaceutically effective amount of silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof; and one or more pharmaceutically acceptable excipients or carriers. [claim 10]
As used herein, the term "single pharmaceutical composition" refers to a dosage form that contains both a) and b) in the same composition.

Thus, a fifth aspect of the present disclosure relates to a kit of parts comprising: i) a first pharmaceutical composition which comprises a pharmaceutically effective amount of a compound selected from the group consisting of a cocrystal of pterostilbene and a coformer as defined above; and pterostilbene or a pharmaceutically acceptable salt thereof; together with one or more pharmaceutically acceptable excipients or carriers; and ii) a second pharmaceutical composition which comprises a pharmaceutically effective amount of silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof; together with one or more pharmaceutically acceptable excipients or carriers; wherein compositions i) and ii) are separate compositions. [claim 11]
For the purposes of the present disclosure, the term "kit-of-parts or package" refers to a combined preparation, wherein pharmaceutical compositions a) and b) are physically separated and are packaged or marked for use together. In the context of the present disclosure "for use together or in combination" does not limit the order in which the pharmaceutical compositions are administered.

A sixth aspect of the present disclosure relates to a combination as defined herein, a single pharmaceutical composition as defined in claim 11, or a kit of parts as defined in claim 12, for use in the treatment of a disease or disorder mediated by the loss of endogenous type I collagen, or for use in joint functionality improvement, articular disease healing; tendon-bone, bone, and muscle repair improvement; or in wound healing. [claim 12]
A seventh aspect of the present disclosure relates to a compound selected from the group consisting of a cocrystal of pterostilbene and a coformer as defined above; and pterostilbene or a pharmaceutically acceptable salt thereof; for use in the treatment of of a disease or disorder mediated by the loss of endogenous type I collagen, or for use in joint functionality improvement, articular disease healing; tendon-bone, bone, and muscle repair improvement; or in wound healing, wherein the compound is to be administered simultaneously, separately or sequentially with silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof.

It also relates to a compound which is silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof for use in the treatment of of a disease or disorder mediated by the loss of endogenous type I collagen, or for use in joint functionality improvement, articular disease healing; tendon-bone, bone, and muscle repair improvement; or in wound healing, wherein the compound is to be administered simultaneously, separately or sequentially with a compound selected from the group consisting of a cocrystal of pterostilbene and a coformer as defined above; and pterostilbene or a pharmaceutically acceptable salt thereof. [claims 13-14]

### Brief Description of Drawings

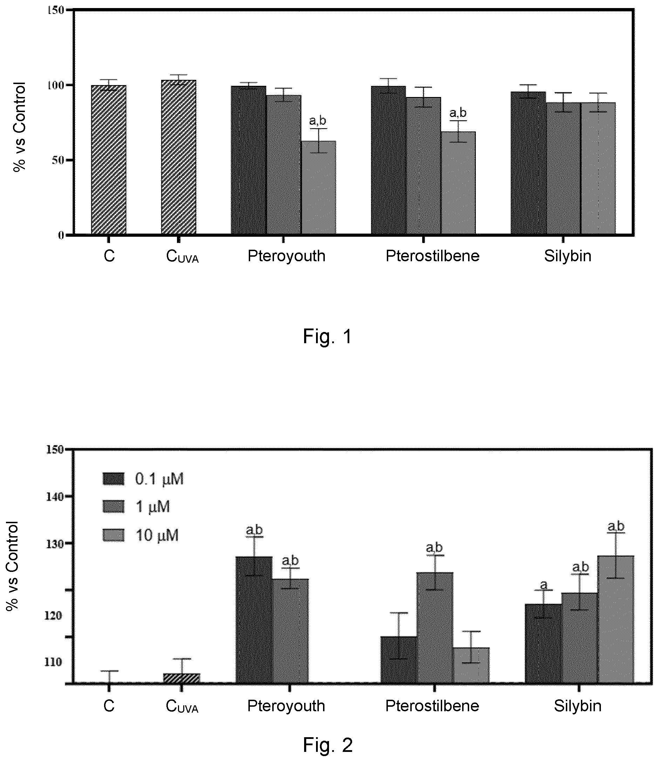

Fig. 1 shows the metabolic activity 7 days after irradiation and treatment with Pteroyouth^{®}, Pterostilbene, or silybin. Shapiro Wilks test was performed to detect parametric or non-parametric populations. ANOVA test was performed to detect statistical differences between pterostilbene vs C and C_{UVA} with Games-Howell as post-hoc, and Kruskal-Wallis with U- Mann-Whitney test was performed to detect statistical differences between Pteroyouth^{®} and silybin vs C and C_{UVA}. ^{a}p< 0.05 vs C; ^{b}p< 0.05 *vs* C_{UVA}.
Fig. 2 shows the total collagen deposition quantification 7 days after irradiation and treatment with Pteroyouth^{®}, Pterostilbene, or silybin. Control was set to 100 %, increased collagen deposition versus Control is shown. Shapiro Wilks test was performed to detect parametric or non-parametric populations. ANOVA test was performed to detect statistical differences between Pteroyouth, silybin and pterostilbene vs C and CUVA with Bonferroni as post-hoc: ^{a}p< 0.05 vs C; ^{b}p< 0.05 vs CUVA. 10 µM Pteroyouth treatment accounted for 97.7 % and 95,6 % of C and C_{UVA} treatments, respectively. No statistically significant differences were found between these treatments.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Thus, as used herein, the singular forms "a", "an", and also the definite article "the" include plural referents unless the context clearly dictates otherwise.

The term "endogenous type I collagen" refers to natural collagen synthesized by the body of an animal, including a human, while "exogenous collagen" refers to a collagen coming from a external source.

As used herein, the term "stereoisomer" refers to all isomers of individual compounds that differ only in the orientation of their atoms in space. The term stereoisomer includes mirror image isomers (enantiomers), mixtures of mirror image isomers (racemates, racemic mixtures), geometric isomers (cis/trans or syn/anti), and isomers that are not mirror images of one another (diastereoisomers). The present invention relates to each of these stereoisomers and also to mixtures thereof.

In general, diastereoisomers and enantiomers can be separated by conventional techniques such as chromatography or fractional crystallization. In particular, enantiomers can be individually obtained using enantiospecific synthesis or can be resolved by conventional techniques of optical resolution to give optically pure isomers.

The term "nutraceutical composition" as used herein include food product, foodstuff, dietary supplement, nutritional supplement, or a supplement composition for a food product or a foodstuff.

As used herein, the term "food product" refers to any food or feed suitable for consumption by humans or animals.

As used herein, the term "foodstuff" refers to any substance fit for human or animal consumption.

The term "dietary supplement" refers to a small amount of a compound for supplementation of a human or animal diet packaged in single or multiple dose units. Dietary supplements can be in form of tablets, capsules, softgels, gelcaps, liquids, powders, bars, drinks, shakes and other food products.

The term "nutritional supplement" refers to a composition comprising a dietary supplement in combination with a source of calories. In some embodiments, nutritional supplements are meal replacements or supplements (e.g., nutrient or energy bars or nutrient beverages or concentrates).

As used herein, the term "cosmetic composition" refers to a composition which is intended to be used to treat, care for or improve the appearance of the skin and/or the scalp. Particular advantageous cosmetic compositions according to the present invention are skin care preparations.

The term "effective amount", as used herein, refers to the minimal amount of a substance and/or agent which is sufficient to achieve a desired and/or required effect.

The expression "pharmaceutically effective amount" as used herein, refers to the amount of a compound that, when administered, is sufficient to prevent development of, or alleviate to some extent, one or more of the symptoms of the disease which is addressed. The particular dose of compound administered according to this disclosure will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity, or other problems or complications commensurate with a reasonable benefit/risk ratio.

The term "pharmaceutically acceptable salt" used herein encompasses any salt formed from pharmaceutically acceptable non-toxic acids including inorganic or organic acids. There is no limitation regarding the salts, except that if used for therapeutic purposes, they must be pharmaceutically acceptable.

The term "cosmetically acceptable" refers to that excipients or carriers suitable for use in contact with human skin without undue toxicity, incompatibility, instability, allergic response, among others.

The expression "nutraceutically acceptable excipients or carriers" refer to acceptable excipients and/or carriers for oral consumption.

The preparation of pharmaceutically acceptable salts of a compound such as silybin (i.e., containing a basic or acidic moiety) can be carried out by methods known in the art. For instance, they can be prepared from the parent compound by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them.

Pterostilbene (trans-3,5-dimethoxy-4-hydroxystilbene) is a natural compound having the following chemical formula:

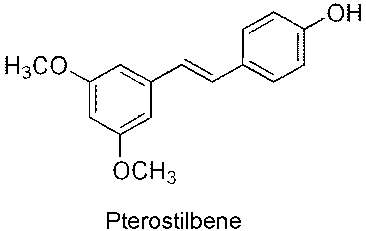

Pterostilbene is known to form cocrystals with some coformers, such as with picolinic acid. Particularly, the cocrystal of pterostilbene and picolinic acid shows a particularly good solubility and bioavailability. Pterostilbene and the cocrystal of pterostilbene and picolinic acid are commercially available. Other cocrystals of pterostilbene can be prepared as disclosed in WO2021123162 or in WO2011097372.

Thus, in an embodiment, the compound for use of the present disclosure is pterostilbene, particularly a pterostilbene cocrystal as defined herein. In a particular embodiment, pterostilbene is in the form of a cocrystal of pterostilbene and picolinic acid, particularly, in the form of the cocrystal of pterostilbene and picolinic acid (1:1). [claim 2]

In all embodiments of the present disclosure referring to pterostilbene, its cocrystals are always contemplated even if they are not specifically mentioned.

Silybin, also known as silibinin (INN), is a mixture of two diastereomers, silybin A and silybin B, in approximately equimolar ratio

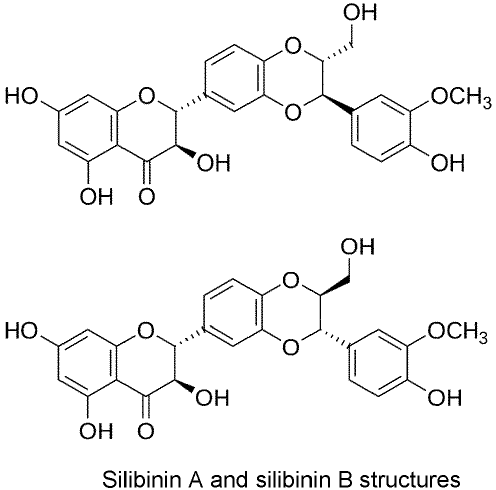

Silybin is commercially available. Silybin can be administered in the form of a water soluble salt, in the form of a complex, or in the form or a glycoside. Silybin may differ from its salts, complexes, or glycosides in some physical properties, but they are equivalent for the purposes of the present disclosure.

Non-limiting examples of pharmaceutically acceptable salts of silybin are silybin inorganic base salts such as silybin sodium salt, silybin potassium salt, or silybin ammonium salt; organic alkali salts such as silybin-N-methylglucamine; or silybin alkaline amino acid salts such as silybin arginine salt, silybin lysinate. Particular examples are silybin meglumine salt, silybin arginine, and silybin sodium salt.

In all embodiments of the present disclosure referring to silybin, its pharmaceutically acceptable salts, or any stereoisomer or mixtures of stereoisomers, either of the compound or of its pharmaceutically acceptable salts, are always contemplated even if they are not specifically mentioned.

Non-limiting examples of pharmaceutically acceptable complexes of silybin include silybin-phosphatidylcholine complex (cf. Kidd P, et al. "A review of the bioavailability and clinical efficacy of milk thistle phytosome: a silybin-phosphatidylcholine complex (Siliphos)" Alternative Medicine Review. 2005, 10 (3): 193-203) and silybin inclusion complex with β-cyclodextrin (cf. Voinovich D, et al. "Solid state mechanochemical activation of Silybum marianum dry extract with betacyclodextrins: Characterization and bioavailability of the coground systems". Journal of Pharmaceutical Sciences. 2009, 98 (11): 4119-29).

Non-limiting examples of pharmaceutically acceptable silybin glycosides (cf. Kosina P, et al. "Antioxidant properties of silybin glycosides". Phytotherapy Research. 2002, 16 (Suppl 1): S33-S39) include silybin galactoside, silybin glucoside, silybin lactoside, and silybin maltoside.

In another embodiment, the compound for use of the present disclosure is silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof. In another embodiment, silybin and it is a mixture of silybin A and silybin B. In another embodiment, In another embodiment, the compound for use of the present disclosure is silybin or a pharmaceutically acceptable salt of thereof, or any stereoisomer or mixtures of stereoisomers of any of them, particularly wherein silybin is a mixture of silybin A and silybin B. [claim 3]

In another embodiment, the disease or disorder mediated by the loss of endogenous type I collagen is selected from the group consisting of a corneal disorder such as corneal ulcer; an articular disorder such as rheumatism, arthritis, degenerative arthritis, and osteoarthritis; and a collagen related inflammatory disease. [claim 4]
As mentioned above, a second aspect of the present disclosure relates to pharmaceutical or cosmetical composition comprising a pharmaceutically or a cosmetically effective amount of a compound as defined herein, together with one or more pharmaceutically or cosmetically acceptable excipients or carriers, for use in the treatment of a disease or disorder mediated by the loss of endogenous type I collagen, or for use in joint functionality improvement, articular disease healing; tendon-bone, bone, and muscle repair improvement; or in wound healing.

Suitable pharmaceutical or cosmetical formulations to be used in the present disclosure are well-known in the art. The election of the pharmaceutical or cosmetical formulation will be determined by the skilled person depending upon the nature of the active compounds present in the composition, and its route of administration. For pharmaceutical compositions, any route of administration, for example, oral, parenteral, or topical administration, can be used.

Thus, in an embodiment, the pharmaceutical composition as defined herein is adapted for oral use, for parenteral use, or for topical use. [claim 8]
The pharmaceutical composition may be formulated for oral administration and may contain one or more physiologically compatible carriers or excipients, in solid or liquid form. For example, formulations suitable for oral administration may include liquid solutions, suspensions, capsules, sachets or tablets, emulsions or dry powdered forms suitable for reconstitution with water.

The pharmaceutical composition may also be formulated for parenteral administration in combination with conventional injectable liquid carriers, such as water or suitable alcohols. Conventional pharmaceutical excipients for injection, such as stabilizing agents, solubilizing agents, and buffers, may be included in such compositions. These pharmaceutical compositions may be injected intramuscularly, intraperitoneally, or intravenously.

Formulations suitable for topical administration include liquid or semi-liquid preparations suitable for penetration through the skin to the site of where treatment is required. Examples of liquid preparations include topical solution or drops (such as eye, ear, or nose drops). Examples of semi-liquid preparations include liniments, lotions, creams, ointment or paste, gel, emugel. Pharmaceutical and cosmetical ingredients are in general those commonly used and generally recognized by person skilled in the art of pharmaceutical and cosmetical formulations.

Throughout the description and claims the word "comprise" and variations of the word, are not intended to exclude other technical features, additives, components, or steps.

Furthermore, the word "comprise" encompasses the case of "consisting of".

The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

### Methods

### Samples

The samples to be tested are provided by CIRCE:
1. Pteroyouth^{®} cocrystal (cocrystal of pterostilbene and picolinic acid, 1:1) from CIRCE Scientific; Batch: CRC-56-002-CAOCBC-01; MW: 360 g/mol; Purity: 66.3 % (as pterostilbene free base).
2. Pterostilbene (CAS No. 537-42-8) provided by CIRCE Scientific; MW: 265 g/mol; Purity: 99.5 %.
3. Silybin (CAS No. 22888-70-6); Batch:2030-08-16 (Glentham Life Sciences); MW: 482 g/mol; Purity: 98 %.

Samples provided by CIRCE were kept at 4 °C. A 10 mM stock solution (expressed as free base) was made in DMSO, aliquoted and kept at -20 °C. For each cell treatment, two different dilutions were made with cell culture media: (1) a 1 mM dilution to prepare the final concentrations of 10, 50, 75 and 100 µM, and (2) a 0.01 mM dilution for the final concentration of 0.1 and 1 µM. All concentrations of Pteroyouth, pterostilbene and silybin in the incubation medium are expressed as free base.

### Cell culture

Immortalized Human Gingival Fibroblasts-hTERT (iHGF) (Applied Biological Materials Inc) were used for the experiments. Cells were grown at 37 °C in an atmosphere of 5% CO₂ using complete fibroblast cell culture medium that consists in Dulbecco's modified Eagle's medium (DMEM) low glucose (Biowest)/Ham's F12 (3/1) (Biowest), supplemented with 10% (v/v) fetal bovine serum embryonic stem cells tested (FBS) (Biowest), 100 g/mL penicillin and 100 g/mL streptomycin (Biowest). Normal gingival tissue contains type I and type III collagens at a ratio of 5:1, which accounts for 99% of the collagen.

### Experimental setup

iHGF were seeded (7 000 cells/well) in 96-well plates (white and transparent tissue culture test-plates) and at confluence, cells were treated with different concentrations (0.1; 1; 10; 50; 75 and 100 µM) of Pteroyouth^{®}, pterostilbene or silybin in complete fibroblast cell culture medium as described above. After 24 h of treatment with test compounds, media was changed to PBS supplemented with 20 mM glucose, and UVA irradiation was performed for 10 minutes at UV-A region (315-400 nm) with 4 bulbs of 40 w lamp at an irradiation distance of 15 cm from the cell monolayer. To measure cell viability, cell culture media and treatments were added again to the cells and metabolic activity was measured 24 h after irradiation. Cells were further cultured for one week, every other day culture media was refreshed with fresh treatments in complete fibroblast cell culture media, as detailed above, supplemented with 100 µM ascorbic acid every other day. Ascorbic acid supplementation is needed to ensure deposition of secreted collagen. Cell viability was determined again at day 7, and cell monolayer was used to quantify collagen deposition. Three independent experiments were performed, with triplicates for each experiment.

### Metabolic activity

Total metabolic activity was evaluated using the PrestoBlue^{™} Cell Viability Reagent (Invitrogen, Catalog Number A13261; Life Technologies, Carlsbad, CA, USA), which is used to measure the metabolic activity of cells, being an indicator of their overall health and functionality. The assay is based on the reduction of resazurin, a blue dye, to resorufin, a pink dye, by metabolically active cells and that can be detected with a plate reader. Thus, cells were incubated with the reagent during 1 h following manufacturer's protocol. Non-treated cells were set as 100%.

### Collagen deposition

Collagen deposition was determined according to Walsh et al 1992. Cell monolayer after 7 days of treatment was washed with PBS and dried overnight at 37 °C in a humidified atmosphere plus 24 hours at 37 °C in a dry atmosphere. Collagen was stained with Sirius red F3BA 0.1% in saturated picric acid for 1 hour. Unbound dye was removed by washing with 10 mM HCl, and dye was solubilized with 100 mM NaOH. Absorbance was measured with a microplate reader at 540 nm. Non-treated cells were set as 100%.

### Statistics

Normality was evaluated through Shapiro-Wilk test. For parametric data, one-way ANOVA and Bonferroni or Games-Howell as post-hoc were performed. For non-parametric data, Kruskal-Wallis with U- Mann-Whitney test was performed. Results were considered statistically significant at p < 0.05. SPSS 27.0 program (SPSS Inc., Chicago, USA) was used for statistical comparison, and GraphPad Prism (version 7, La Jolla, CA, USA) was used to represent the data.

### Results

### Metabolic activity

Metabolic activity was evaluated 24 hours after cell irradiation of cells pretreated for 24 hours with different concentrations (0.1; 1; 10; 50; 75 and 100 µM) of Pteroyouth^{®}, pterostilbene or silybin as an indicator of cell viability. Further determinations were only performed in those groups with a cell viability >70 %.

Similar results were observed after 7 days of culture and treatment (that was refreshed with every media change 3 times/week) in the metabolic activity
Thus, as shown by the results (Figure 2), Pteroyouth and pterostilbene at concentrations of 0.1 and 1 µM, and silybin at concentrations of 0.1, 1 and 10 µM were demonstrated to maintain cell viability after UV irradiation.

### Total collagen deposition

Total collagen deposition was evaluated 7 days after cell irradiation and treatment with different concentrations (0.1; 1; 10 µM) of Pteroyouth^{®}, pterostilbene or silybin. As seen in Figure 2, higher collagen deposition was induced by treatment with 0.1 and 1 µM Pteroyouth^{®}, 1 µM pterostilbene and 0.1, 1 and 10 µM silybin, compared to both C and CuVA.

As shown in the results, Pteroyouth at concentrations of 0.1 and 1 µM, 1 µM pterostilbene, and silybin at concentrations of 0.1, 1 and 10 µM promoted total collagen deposition in human fibroblasts.

### Citation List

1. WO2021123162.
2. WO2011097372
3. Kidd P, et al. "A review of the bioavailability and clinical efficacy of milk thistle phytosome: a silybin-phosphatidylcholine complex (Siliphos)" Alternative Medicine Review. 2005, 10 (3): 193-203.
4. Voinovich D, et al. "Solid state mechanochemical activation of Silybum marianum dry extract with betacyclodextrins: Characterization and bioavailability of the coground systems". Journal of Pharmaceutical Sciences. 2009, 98 (11): 4119-29.
5. Kosina P, et al. "Antioxidant properties of silybin glycosides". Phytotherapy Research. 2002, 16 (Suppl 1): S33-S39.
6. Walsh BJ, Thornton SC, Penny R, Breit SN. Microplate reader-based quantitation of collagens. Anal Biochem. 1992; 203(2):187-90. doi: 10.1016/0003- 2697(92)90301-m.

## Claims

1. A compound selected from the group consisting of:
- a cocrystal of pterostilbene and a coformer selected from the group consisting of picolinic acid, 1,4-dimethylpiperazine, 2,3,5-trimethylpyrazine, theophylline, ethylenediamine, 1,4-diazabicyclo[2.2.2]octane (DABCO), and 1,4,8,11-tetrazacyclohexandecane, 2,4-dihydroxybenzoic acid (2,4-DHBA), indole, lysine, orotic acid, phenanthroline, urea, L-arginine, glycine, caffeine, carbamazepine, piperazine, and glutaric acid;
- pterostilbene or a pharmaceutically acceptable salt thereof;
- silybin or a pharmaceutically acceptable salt of thereof, or any stereoisomer or mixtures of stereoisomers of any of them; a complex of silybin or of any of stereoisomer or mixtures of stereoisomers thereof; or a glycoside of silybin or of any stereoisomer or mixtures of stereoisomers thereof;
either for use in the treatment of a disease or disorder mediated by the loss of endogenous type I collagen, or for use in joint functionality improvement, articular disease healing; tendon-bone, bone, and muscle repair improvement; or in wound healing.

2. The compound for use of claim 1, wherein the compound is cocrystal of pterostilbene and picolinic acid.

3. The compound for use of claim 1, wherein the compound is silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof; particularly wherein silybin is a mixture of silybin A and silybin B.

4. The compound for use of any one of claims 1 to 3, wherein the disease or disorder mediated by the loss of endogenous type I collagen is selected from the group consisting of a corneal disorder such as corneal ulcer; an articular disorder such as rheumatism, arthritis, degenerative arthritis, and osteoarthritis; and a collagen related inflammatory disease.

5. The compound for use of any one of claims 1 to 4, wherein the compound is to be administered in the form of a pharmaceutical composition, a cosmetical composition, or a nutraceutical composition..

6. A pharmaceutical, cosmetical, or nutraceutical composition comprising a pharmaceutically, a cosmetically, or a nutraceutically effective amount of a compound as defined in any one of claims 1 to 3, together with one or more pharmaceutically, cosmetically, or nutraceutically acceptable excipients or carriers, for use in the treatment of a disease or disorder mediated by the loss of endogenous type I collagen, or for use in joint functionality improvement, articular disease healing; tendon-bone, bone, and muscle repair improvement; or in wound healing.

7. The pharmaceutical, cosmetical, or nutraceutical composition for use of claim 6, wherein the disease or disorder mediated by the loss of endogenous type I collagen is selected from the group consisting of a corneal disorder such as corneal ulcer; an articular disorder such as rheumatism, arthritis, degenerative arthritis, and osteoarthritis; and a collagen related inflammatory disease

8. The pharmaceutical composition according to claims 6 or 7, which is adapted for oral use, for parenteral use, or for topical use.

9. A combination comprising:
a) a compound selected from the group consisting of:
- a cocrystal of pterostilbene and a coformer selected from the group consisting of picolinic acid, 1,4-dimethylpiperazine, 2,3,5-trimethylpyrazine, theophylline, ethylenediamine, 1,4-diazabicyclo[2.2.2]octane (DABCO), and 1,4,8,11-tetrazacyclohexandecane, 2,4-dihydroxybenzoic acid (2,4-DHBA), indole, lysine, orotic acid, phenanthroline, urea, L-arginine, glycine, caffeine, carbamazepine, piperazine, and glutaric acid;
- pterostilbene or a pharmaceutically acceptable salt thereof; and
b) silybin or a pharmaceutically acceptable salt of thereof, or any stereoisomer or mixtures of stereoisomers of any of them; a complex of silybin or of any of stereoisomer or mixtures of stereoisomers thereof; or a glycoside of silybin or of any stereoisomer or mixtures of stereoisomers thereof.

10. A single pharmaceutical composition which comprises:
a) a pharmaceutically effective amount of a compound selected from the group consisting of a cocrystal of pterostilbene and a coformer selected from the group consisting of picolinic acid, 1,4-dimethylpiperazine, 2,3,5-trimethylpyrazine, theophylline, ethylenediamine, 1,4-diazabicyclo[2.2.2]octane (DABCO), and 1,4,8,11-tetrazacyclohexandecane, 2,4-dihydroxybenzoic acid (2,4-DHBA), indole, lysine, orotic acid, phenanthroline, urea, L-arginine, glycine, caffeine, carbamazepine, piperazine, and glutaric acid; and pterostilbene or a pharmaceutically acceptable salt thereof;
b) a pharmaceutically effective amount of silybin or a pharmaceutically acceptable salt of thereof, or any stereoisomer or mixtures of stereoisomers of any of them; a complex of silybin or of any of stereoisomer or mixtures of stereoisomers thereof; or a glycoside of silybin or of any stereoisomer or mixtures of stereoisomers thereof;
and one or more pharmaceutically acceptable excipients or carriers.

11. A kit of parts comprising:
i) a first pharmaceutical composition which comprises a pharmaceutically effective amount of a compound selected from the group consisting of a cocrystal of pterostilbene and a coformer selected from the group consisting of picolinic acid, 1,4-dimethylpiperazine, 2,3,5-trimethylpyrazine, theophylline, ethylenediamine, 1,4-diazabicyclo[2.2.2]octane (DABCO), and 1,4,8,11-tetrazacyclohexandecane, 2,4-dihydroxybenzoic acid (2,4-DHBA), indole, lysine, orotic acid, phenanthroline, urea, L-arginine, glycine, caffeine, carbamazepine, piperazine, and glutaric acid; and pterostilbene or a pharmaceutically acceptable salt thereof; together with one or more pharmaceutically acceptable excipients or carriers; and
ii) a second pharmaceutical composition which comprises a pharmaceutically effective amount of ssilybin or a pharmaceutically acceptable salt of thereof, or any stereoisomer or mixtures of stereoisomers of any of them; a complex of silybin or of any of stereoisomer or mixtures of stereoisomers thereof; or a glycoside of silybin or of any stereoisomer or mixtures of stereoisomers thereof; together with one or more pharmaceutically acceptable excipients or carriers;
wherein compositions i) and ii) are separate compositions.

12. A combination as defined in claim 9, a single pharmaceutical composition as defined in claim 10, or a kit of parts as defined in claim 11, for use in the treatment of a disease or disorder mediated by the loss of endogenous type I collagen, or for use in joint functionality improvement, articular disease healing; tendon-bone, bone, and muscle repair improvement; or in wound healing.

13. A combination, a single pharmaceutical composition, or a kit of parts as defined in claim 12, wherein the disease or disorder mediated by the loss of endogenous type I collagen is selected from the group consisting of a corneal disorder such as corneal ulcer; an articular disorder such as rheumatism, arthritis, degenerative arthritis, and osteoarthritis; and a collagen related inflammatory disease.

14. A compound selected from the group consisting of a cocrystal of pterostilbene and a coformer selected from the group consisting of picolinic acid, 1,4-dimethylpiperazine, 2,3,5-trimethylpyrazine, theophylline, ethylenediamine, 1,4-diazabicyclo[2.2.2]octane (DABCO), and 1,4,8,11-tetrazacyclohexandecane, 2,4-dihydroxybenzoic acid (2,4-DHBA), indole, lysine, orotic acid, phenanthroline, urea, L-arginine, glycine, caffeine, carbamazepine, piperazine, and glutaric acid; and pterostilbene or a pharmaceutically acceptable salt thereof;
for use in the treatment of a disease or disorder mediated by the loss of endogenous type I collagen, or for use in joint functionality improvement, articular disease healing; tendon-bone, bone, and muscle repair improvement; or in wound healing,
wherein the compound is to be administered simultaneously, separately or sequentially with silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof.

15. A compound selected from the group consisting of silybin or a pharmaceutically acceptable salt of thereof, or any stereoisomer or mixtures of stereoisomers of any of them; a complex of silybin or of any of stereoisomer or mixtures of stereoisomers thereof, or a glycoside of silybin or of any stereoisomer or mixtures of stereoisomers thereof;
for use in the treatment of a disease or disorder mediated by the loss of endogenous type I collagen, or for use in joint functionality improvement, articular disease healing; tendon-bone, bone, and muscle repair improvement; or in wound healing,
wherein the compound is to be administered simultaneously, separately or sequentially with a compound selected from the group consisting of a cocrystal of pterostilbene and a coformer selected from the group consisting of picolinic acid, 1,4-dimethylpiperazine, 2,3,5-trimethylpyrazine, theophylline, ethylenediamine, 1,4-diazabicyclo[2.2.2]octane (DABCO), and 1,4,8,11-tetrazacyclohexandecane, 2,4-dihydroxybenzoic acid (2,4-DHBA), indole, lysine, orotic acid, phenanthroline, urea, L-arginine, glycine, caffeine, carbamazepine, piperazine, and glutaric acid; and pterostilbene or a pharmaceutically acceptable salt thereof.
